# EUROPÄISCHE PATENTSCHRIFT

(11) **EP 0 999 822 B1**
(45) Veröffentlichungstag und Bekanntmachung des Hinweises auf die Patenterteilung: **15.09.2004**
(21) Anmeldenummer: 98934954.3
(22) Anmeldetag: 10.06.1998
(51) Int. Cl.: A61K 7/06, A61K 7/50

(54) **WÄSSRIGE PERLGLANZKONZENTRATE**
AQUEOUS PEARLY LUSTRE CONCENTRATES
CONCENTRES AQUEUX NACRES

(30) Priorität: 19.06.1997 DE 19725964
(43) Veröffentlichungstag der Anmeldung: 17.05.2000
(73) Patentinhaber: Cognis Deutschland GmbH & Co. KG, 40589 Düsseldorf (DE)
(72) Erfinder: ANSMANN, Achim, D-40699 Erkrath (DE); FABRY, Bernd, D-41352 Korschenbroich (DE); KAWA, Rolf, D-40789 Monheim (DE)
(86) Internationale Anmeldenummer: PCT/EP1998/003545
(87) Internationale Veröffentlichungsnummer: WO 1998/058621

(56) Entgegenhaltungen:
- EP-A- 0 500 941
- CHEMICAL ABSTRACTS, vol. 121, no. 18, 31. Oktober 1994 Columbus, Ohio, US; abstract no. 212617, N.MATSUDA E.A.: "Transparent hair rinses containing condensed castor oil fatty acid amidipropyl betaines" XP002080915 & JP 05 331028 A (IWASE COSFA) 14. Dezember 1993
- CHEMICAL ABSTRACTS, vol. 121, no. 8, 22. August 1994 Columbus, Ohio, US; abstract no. 91298, N.MATSUDA E.A.: "Transparent shampoo-hair rinse containing condensed castor oil fatty acid amidopropyl betaines" XP002080916 & JP 05 331027 A (IWASE COSFA) 14. Dezember 1993

## Beschreibung

### Gebiet der Erfindung

Die Erfindung betrifft wäßrige Perlglanzkonzentrate mit einem Gehalt an ausgewählten Hydroxyverbindungen. Emulgatoren und gegebenenfalls Polyolen, ein Verfahren zu ihrer Herstellung, ein weiteres Verfahren zur Herstellung von perlglänzenden oberflächenaktiven Zubereitungen unter Verwendung der Konzentrate sowie die Verwendung der Hydroxyverbindungen als Perlglanzwachse.

### Stand der Technik

Der weich schimmernde Glanz von Perlen hat auf den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind. Perlglanz zu erzeugen. Für die moderne Kosmetik sind hingegen Perlglanzwachse, insbesondere vom Typ der Glycolmono- und -difettsäureester von Bedeutung, die überwiegend zur Erzeugung von Perlglanz in Haarshampoos und Duschgelen eingesetzt werden. Eine Übersicht zu modernen, perlglänzenden Formulierungen findet sich von A.Ansmann und R.Kawa in **Parf.Kosm. 75, 578 (1994).**

Der Stand der Technik kennt eine Vielzahl von Formulierungen, die oberflächenaktiven Mitteln den gewünschten Perlglanz verleihen. So sind beispielsweise aus den beiden Deutschen Patentanmeldungen **DE-A1 3843572** und **DE-A1 4103551** (Henkel) Perlglanzkonzentrate in Form fließfähiger wäßriger Dispersionen bekannt, die 15 bis 40 Gew.-% perlgiänzender Komponenten, 5 bis 55 Gew.-% Emulgatoren und 0.1 bis 5 bzw. 15 bis 40 Gew.-% Polyole enthalten. Bei den Perlglanzwachsen handelt es sich um acylierte Polyalkylenglycole, Monoaikanolamide, lineare, gesättigte Fettsäuren oder Ketosulfone. In den beiden Europäischen Patentschriften **EP-B1 0181773** und **EP-B1 0285389** (Procter & Gamble) werden Shampoozusammensetzungen vorgeschlagen, die Tenside. nicht-flüchtige Silicone und Perlglanzwachse enthalten. Gegenstand der Europäischen Patentanmeldung **EP-A2 0205922** (Henkel) sind fließfähige Perlglanzkonzentrate, die 5 bis 15 Gew.-% acylierte Polyglycole, 1 bis 6 Gew.-% Fettsäuremonoethanolamide und 1 bis 5 Gew.-% nichtionische Emulgatoren enthalten. Gemäß der Lehre der Europäischen Patentschrift **EP-B1 0569843** (Hoechst) lassen sich nichtionische, fließfähige Pengfanzdispersionen auch erhalten, indem man Mischungen von 5 bis 30 Gew.-% acylierten Polyglycolen und 0,1 bis 20 Gew.-% ausgewählten nichtionischen Tensiden herstellt Aus der Europäischen Patentanmeldung **EP-A2 0581193** (Hoechst) sind femer fließfähige, konservierungsmittelfreie Perlglanzdispersionen bekannt, die acylierte Polyglycolether, Betaine, Aniontenside und Glycerin enthalten. Schließlich wird in der Europäischen Patentanmeldung **EP-A1 0684302** (Th.Goldschmidt) die Verwendung von Polyglycerinestern als Kristallisationshilfsmittel für die Herstellung von Perlglanzkonzentraten vorgeschlagen.

Die EP-A1 0500 941 offenbart Zubereitungen enthaltend Cocobelaine, 12-Hydroxystearinsäure und Propylenglykol. Gegenstand der JP 05-331028 A und JP 05- 331027 A sind Zubereitungen enthaltend 12-Hydroxystearinsäureamidopropylbetain in Kombination mit Tensiden.

Trotz der Vielzahl von Mitteln besteht im Markt ein ständiges Bedürfnis nach neuen Perlglanzwachsen, die beispielsweise im Gegensatz zu acylierten Polyglycolen keine Ethylenoxideinheiten aufweisen und sich gegenüber den Produkten des Stands der Technik auch bei verminderter Einsatzmenge durch einen brillanten Glanz auszeichnen, die die Mitverwendung kritischer Inhaltsstoffe wie beispielsweise von Siliconen zulassen, ohne daß die Stabilität der Formulierungen beeinträchtigt wird, gleichzeitig über Estergruppen verfügen, damit eine ausreichende biologische Abbaubarkeit gewährleistet ist und die insbesondere in konzentrierter Form noch leicht beweglich und damit handhabbar sind. Die Aufgabe der vorliegenden Erfindung hat somit darin bestanden, neue Perlglanzkonzentrate mit dem geschilderten komplexen Anforderungsprofil zur Verfügung zu stellen.

### Beschreibung der Erfindung

Gegenstand der Erfindung sind wäßrige Perlglanzkonzentrate, enthaltend - bezogen auf den nicht-wäßrigen Anteil -
(a) 1 bis 99,9 Gew.-% 12-Hydroxystearinsäure bzw. deren Salze und/oder 12-Hydroxystearyl-alkohol,
(b) 0,1 bis 90 Gew.-% Emulgatoren, die ausgewählt sind aus der Gruppe, die gebildet wird von:
   (b1) Anlagerungsprodukten von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Triglyceride;
   (b2) C_{12/18}-Fettsäuremono- und -diestem von Anlagerungsprodukten von 1 bis 30 Mol Ethylen-oxid an Glycerin;
   (b3) Glycerinmono- und -diesem und Sorbitanmono- und -diestern von gesättigten und unge-sättigten Fettsäuren mit 6 bis 22 Kohlenstoffatomen und deren Ethylenoxidanlage-rungsprodukte;
   (b4) Alkylmono- und -oligoglycasiden mit 8 bis 22 Kohlenstoffatomen im Alkylrest und deren ethoxylierten Analoga;
   (b5) Anlagerungsprodukten von 15 bis 60 Mol Ethylenoxid an Ricinusöl und/oder gehärtetes Ricinusöl;
   (b6) Polyolestern;
   (b7) Anlagerungsprodukten von 2 bis 15 Mol Ethylenoxid an Ricinusöl und/oder gehärtetes Ricinusöl;
   (b8) Partialestern auf Basis linearer, verzweigter, ungesättigter bzw. gesättigter C_{12/22}-Fett-säuren, Ricinolsäure sowie 12-Hydroxystearinsäure und Glycerin, Polyglycerin, Penta-erythrit, Dipentaerythrit, Zuckeralkoholen sowie Polyglucosiden;
   (b9) Trialkylphosphaten;
   (b10) Wollwachsalkoholen;
   (b11) Polysiloxan-Polyalkyl-Polyether-Copolymeren bzw. entsprechenden Derivaten;
   (b12) Mischestem aus Pentaerythrit, Fettsäuren, Citronensäure und Fettalkoholen;
   (b13) Polyalkylenglycolen.
(c) 0 bis 40 Gew.-% Polyole,
mit der Maßgabe, daß sich die Mengenangaben zu 100 Gew.-% ergänzen.

Überraschenderweise wurde gefunden, daß die genannten Hydroxyverbindungen ausgezeichnete perlglänzende Eigenschaften besitzen und sich gegenüber den Produkten des Stands der Technik durch eine höhere Brillanz bei geringerer Einsatzmenge, besondere Feinteiligkeit und Lagerstabilität auszeichnen. Die Perigianzwachse sind leicht biologisch abbaubar. in konzentrierter Form dünnffüssig und erlauben auch die Einarbeitung von problematischen Inhaltsstoffen wie beispielsweise Siliconen in kosmetische Zubereitungen.

### Hydroxyverbindungen

12-Hydroxystearinsäure und 12-Hydroxystearylalkohol stellen bekannte Handelsprodukte dar, die üblicherweise durch Härtung, d.h. Absättigung der entsprechenden ungesättigten homologen Ricinolsäure und Ricinylalkohol. hergestellt werden. Als Salze kommen die Alkali- und Erdalkalisalze, namentlich die Natrium-, Kalium-. Magnesium- oder Calciumsalze sowie die Aluminium- und Zinksalze in Frage.

### Emulgatoren

Die erfindungsgemäßen Perlglanzkonzentrate können als Emulgatoren nichtionogene Tenside aus mindestens einer der folgenden Gruppen enthalten:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Triglyceride;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit. Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte. dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt

C_{8/18}-Alkylmono- und oligoglycoside, ihre Herstellung und ihre Verwendung als oberflächenaktive Stoffe sind literaturbekannt Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als zusätzliche Emulgatoren zwitterionische Tenside verwendet werden. Als zwitterionische Tenside werden solche oberflächenaktiven Verbindungen bezeichnet die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Afkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampho-lytische Tenside sind N-Alkylglycine, N-Alkylpropiansäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze. besonders bevorzugt sind.

Die erfindungsgemäßen Perlglanzkonzentrate können die Emulgatoren in Mengen von 0,1 bis 90, vorzugsweise 5 bis 50 und insbesondere 10 bis 40 Gew.-% enthalten.

### Polyole

Polyole, die im Sinne der Erfindung als Komponente (c) in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol. Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Die erfindungsgemäßen Perlglanzkonzentrate können die Polyole, vorzugsweise Glycerin, Propylenglycol, Butylenglycol, Hexylenglycol sowie Polyethylenglycole mit einem durchschnittlichen Molekulargewicht im Bereich von 100 bis 1.0000 in Mengen von 0,1 bis 40, vorzugsweise 0,5 bis 15 und insbesondere 1 bis 5 Gew.-% enthalten.

### Herstellverfahren

In einer bevorzugten Ausführungsform, die ebenfalls Gegenstand der Erfindung ist, erfolgt die Herstellung der Perlglanzkonzenfrate. indem man eine Mischung aus den Komponenten (a), (b) und (c) herstellt, auf eine Temperatur erwärmt, die 1 bis 30°C oberhalb des Schmelzpunktes der Mischung liegt, mit der erforderlichen Menge Wasser etwa der gieichen Temperatur mischt und anschließend auf Raumtemperatur abkühlt. Ferner ist es möglich, eine konzentrierte wäßrige (Anion-)Tensidpaste vorzulegen, das Perlglanzwachs in der Wärme einzurühren und die Mischung anschließend mit weiterem Wasser auf die gewünschte Konzentration zu verdünnen oder das Vermischen in Gegenwart polymerer hydrophiler Verdickungsmittel wie etwa Hydroxypropylcellulosen, Xanthan Gum oder Polymeren vom Carbomer-Typ durchzuführen.

### Gewerbliche Anwendbarkeit

Die erfindungsgemäßen Perlglanzkonzentrate eignen sich zur Einstellung einer Trübung in oberflächenaktiven Zubereitungen wie beispielsweise Haarshampoos oder manuellen Geschirrspülmitteln. Ein weiterer Gegenstand der Erfindung betrifft daher ein Verfahren zur Herstellung getrübter und perlglänzender flüssiger, wäßriger Zubereitungen wasserlöslicher grenzflächenaktiver Stoffe, bei dem man den klaren wäßrigen Zubereitungen bei 0 bis 40°C die Perlglanzkonzentrate in einer Menge von 0,5 bis 40, vorzugsweise 1 bis 20 Gew.-% der Zubereitung zusetzt und unter Rühren darin verteilt.

### Tenside

Die oberflächenaktiven Zubereitungen, die in der Regel einen nicht-wäßrigen Anteil im Bereich von 1 bis 50 und vorzugsweise 5 bis 35 Gew.-% aufweisen, können nichtionische, anionische, kationische und/oder amphotere bzw. amphotere Tenside enthalten, deren Anteil an den Mitteln üblicherweise bei etwa 50 bis 99 und vorzugsweise 70 bis 90 Gew.-% beträgt. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle. vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine. Alkylamidobetaine, Aminopropionate, Aminoglycinate, Imidazoliniumbetaine und Sulfobetaine, Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineraiöladditive", Thieme Verlag, Stuttgart, 1978, S. 123-217** verwiesen. Die gleichen Tenside können auch unmittelbar zur Herstellung der Perlglanzkonzentrate eingesetzt werden. die anionischen Tenside eignen sich auch als Emulgatoren. In diesem Zusammenhang ist der Einsatz von Alkylethersulfaten als anionische Emulgatoren bevorzugt.

### Hilfs- und Zusatzstoffe

Die oberflächenaktiven Zubereitungen, denen die erfindungsgemäßen Perlglanzkonzentrate zugesetzt werden, können weitere Hilfs- und Zusatzstoffe enthalten wie beispielsweise Ölkörper, weitere Perlglanzwachse, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen. insbesondere 2-Ethylhexanol. Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

Als weitere **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierten Carbonsäuren mit Fettalkoholen mit 6 bis 22 Kohlenstoffatomen, speziell langkettige Ester der Weinsäure; Fettstoffe wie beispielsweise Fettalkohole, Fettketone, Fettaldehyde. Fettether und Fettcarbonate, die in Summe mindestens 24 Kohlenstoffatome aufweisen, speziell Lauron und Distearylether: Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen: sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden. wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglücamiden gleicher Kettenlänge und/oder Polyglycerinpoly-12-hydroxystearaten. Geeignete Verdickungsmittel sind beispielsweise Polysaccharide, insbesondere Xanthan-Gum, Guar-Guar, Agar-Agar, Alginate und Tylosen. Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan. Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylenirimamin (Cartaretine®, Sandoz/CH), Polyaminopolyamide wie z.B. beschrieben in der **FR-A 22 52 840** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar@ C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete Siliconverbindungen sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs. Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol. Panthenol, AHA-Säuren, Pflanzenextrakte und Vitaminkompiexe zu verstehen. Als **Antischuppenmittel** können Climbazol. Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate. Kollagen, Hyaluronsäure bzw. deren Salze und ähnliche Verbindungen. Zur Verbesserung des Fließverhaltens können ferner Hydrotrope wie beispielsweise Ethanol, Isopropylalkohol, Propylenglycol oder Glucose eingesetzt werden. Als Konservierungsmittel eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%. bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen: vorzugsweise arbeitet man nach der Phaseninversionstemperatur-Methode.

Ein letzter Gegenstand der Erfindung betrifft schließlich die Verwendung von 12-Hydroxystearinsäure bzw. deren Salze und/oder 12-Hydroxystearylalkohol zur Herstellung von oberflächenaktiven Zubereitungen.

### Beispiele

Die erfindungsgemäßen Perlglanzkonzentrate 1 bis 6 sowie die Vergleichsmischungen V1 und V2 wurden 14 Tage bei 40°C gelagert und die Viskosität nach der Brookfield-Methode in einem RVT-Viskosimeter (23°C, 10 Upm, Spindel 5) bestimmt. Anschließend wurden wäßrige Haarshampooformulierungen durch Vermischen der Einsatzstoffe bei 20°C zubereitet, die jeweils 2 g der Perlglanzkonzentrate, 15 g Kokosfettalkohol+2EO-sulfat-Natriumsalz, 3 g Dimethylpolysiloxan, 5 g Kokosalkyllucosid und 1,5 g eines Esterquats (Wasser ad 100 Gew.-%) enthielten. Die Feinteiligkeit der Perlglanzkristalle in den Haarshampoos wurde unter dem Mikroskop visuell auf einer Skala von 1 = sehr feine Kristalle bis 5 = grobe Kristalle beurteilt. Die Beurteilung des Perlglanzes erfolgte ebenfalls auf einer Skala von 1 = brillant bis 5 = stumpf; die Trübung wurde visuell bestimmt und mit (+) = trüb oder (-) = trübungsfrei beurteilt. Die Zusammensetzungen und Ergebnisse sind in Tabelle 1 zusammengefaßt: alle Mengenangaben verstehen sich als Gew.-%

**Tabelle 1**

| **Zusammensetzung und Performance von Periglanzkonzentraten** | | | | | | | | |
|---|---|---|---|---|---|---|---|---|
| **Zusammensetzung** | **1** | **2** | **3** | **4** | **5** | **6** | **V1** | **V2** |
| 12-Hydroxystearinsäure | 25 | - | 20 | 20 | 20 | 20 | - | - |
| 12-Hydroxystearylalkohol | - | 25 | - | - | - | - | - | - |
| Ethylenglycoldistearat | - | - | 5 | - | - | - | 25 | - |
| Kokosfettsäurediethanolamid | - | - | - | 5 | - | - | - | - |
| Stearinsäuremonoglycerid | - | - | - | - | 5 | - | - | - |
| Distearylether | - | - | - | - | - | 5 | - | - |
| Stearinsäure | - | - | - | - | - | - | - | 25 |
| Kokosalkohol+4EO | 5 | 5 | 5 | - | 5 | 5 | 5 | 5 |
| Kokosalkylglucosid | 9 | 9 | 9 | 15 | 9 | 9 | 9 | 9 |
| Kokosfettsäurebetain | 5 | 5 | 5 | 4 | 5 | 5 | 5 | 5 |
| Glycerin | 5 | 5 | 5 | 5 | 5 | 5 | 5 | 5 |
| Wasser | ad 100 | | | | | | | |

| ***Viskosität der Konzentrate [mPas]*** | | | | | | | | |
|---|---|---|---|---|---|---|---|---|
| - nach 1 d, 40°C | 8.000 | 8.300 | 7.400 | 7.800 | 8.200 | 8.200 | 9.500 | 9.300 |
| - nach 14 d, 40°C | 7.900 | 7.900 | 6.900 | 7.700 | 7.900 | 7.900 | 7.200 | 8.100 |

| ***Perlglanz in der Formulierung*** | | | | | | | | |
|---|---|---|---|---|---|---|---|---|
| - Brillanz | 1,5 | 1,5 | 1,0 | 1,0 | 1,5 | 1,5 | 2,0 | 3,0 |
| - Feinteiligkeit | 1,5 | 1,5 | 1,5 | 2,0 | 1,5 | 1,0 | 3,0 | 3,0 |
| - Trübung | - | - | - | - | - | - | + | + |

## Patentansprüche

1. Wäßrige Perlglanzkonzentrate, enthaltend - bezogen auf den nicht-wäßrigen Anteil -
(a) 1 bis 99,9 Gew.-% 12-Hydroxystearinsäure bzw. deren Salze und/oder 12-Hydroxystearylalkohol,
(b) 0,1 bis 90 Gew.-% Emulgatoren, die ausgewählt sind aus der Gruppe, die gebildet wird von:
(b1) Anlagerungsprodukten von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Triglyceride;
(b2) C_{12/18}-Fettsäuremono- und -diestem von Anlagerungsprodukten von 1 bis 30 Mol Ethylenoxid an Glycerin;
(b3) Glycerinmono- und -diestem und Sorbitanmono- und -diestem von gesättigten und ungesättigten Fettsäuren mit 6 bis 22 Kohlenstoffatomen und deren Ethylenoxidanlagerungsprodukte;
(b4) Alkylmono- und -oligoglycosiden mit 8 bis 22 Kohlenstoffatomen im Alkylrest und deren ethoxylierten Analoga;
(b5) Anlagerungsprodukten von 15 bis 60 Mol Ethylenoxid an Ricinusöl und/oder gehärtetes Ricinusöl;
(b6) Polyolestern;
(b7) Anlagerungsprodukten von 2 bis 15 Mol Ethylenoxid an Ricinusöl und/oder gehärtetes Ricinusöl;
(b8) Partialestem auf Basis linearer, verzweigter, ungesättigter bzw. gesättigter G_{12/22}-Fettsäuren, Ricinolsäure sowie 12-Hydroxystearinsäure und Glycerin, Potyglycerin, Pentaerythrit, Dipentaerythrit, Zuckeralkoholen sowie Polyglucosiden;
(b9) Trialkylphosphaten;
(b10) Wollwachsalkoholen;
(b11) Polysiloxan-Polyalkyl-Polyether-Copolymeren bzw. entsprechenden Derivaten;
(b12) Mischestem aus Pentaerythrit, Fettsäuren, Citronensäure und Fettalkoholen;
(b13) Polyalkylenglycolen.
(c) 0 bis 40 Gew.-% Polyole,
mit der Maßgabe, daß sich die Mengenangaben zu 100 Gew.-% ergänzen.

2. Pedglanzkonzentrate nach Anspruch 1, **dadurch gekennzeichnet, daß** sie zusätzlich Emulgatoren vom Typ der Alkylethersulfate enthalten.

3. Perlglanzkonzentrate nach den Ansprüchen 1 bis 2, **dadurch gekennzeichnet, daß** sie als Komponente (c) 0,1 bis 40 Gew.-% Glycerin, 1,2-Propylenglycol, Butylenglycol, Hexylenglycol und/oder Polyethylenglycole mit einem durchschnittlichen Molekulargewicht im Bereich von 100 bis 1.000 Dalton enthalten.

4. Verfahren zur Herstellung von Perlglanzkonzentraten nach Anspruch 1, **dadurch gekennzeichnet, daß** man eine Mischung aus den Komponenten (a), (b) und (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt.

5. Verfahren zur Herstellung getrübter und perlglänzender flüssiger, wäßriger Zubereitungen wasserlöslicher grenzflächenaktiver Stoffe, bei dem man den klaren wäßrigen Zubereitungen bei 0 bis 40°C Perlglanzkonzentrate nach den Ansprüchen 1 bis 3 in einer Menge von 0,5 bis 40 Gew.-% der Zubereitung zusetzt und unter Rühren darin verteilt.

6. Verwendung von 12-Hydroxystearinsäure bzw. deren Salze und/oder 12-Hydroxystearylalkohol als Perlglanzwachse zur Herstellung von oberflächenaktiven Zubereitungen.

## Claims

1. Aqueous pearlizing concentrates containing - based on the nonaqueous component -
(a) 1 to 99.9% by weight of 12-hydroxystearic acid or salts thereof and/or 12-hydroxystearyl alcohol,
(b) 0.1 to 90% by weight of emulsifiers selected from the group consisting of
(b1) products of the addition of 2 to 30 mol ethylene oxide and/or 0 to 5 mol propylene oxide onto linear fatty alcohols containing 8 to 22 carbon atoms, onto fatty acids containing 12 to 22 carbon atoms, to alkylphenols containing 8 to 15 carbon atoms in the alkyl group and onto triglycerides;
(b2) C_{12/18} fatty acid monoesters and diesters of addition products of 1 to 30 mol ethylene oxide onto glycerol;
(b3) glycerol monoesters and diesters and sorbitan monoesters and diesters of saturated and unsaturated fatty acids containing 6 to 22 carbon atoms and ethylene oxide adducts thereof;
(b4) alkyl mono- and oligoglycosides containing 8 to 22 carbon atoms in the alkyl group and ethoxylated analogs thereof;
(b5) products of the addition of 15 to 60 mol ethylene oxide onto castor oil and/or hydrogenated castor oil;
(b6) polyol esters;
(b7) products of the addition of 2 to 15 mol ethylene oxide onto castor oil and/or hydrogenated castor oil;
(b8) partial esters based on linear, branched, unsaturated or saturated C_{12/22} fatty acids, ricinoleic acid and also 12-hydroxystearic acid and glycerol, polyglycerol, pentaerythritol, dipentaerythritol, sugar alcohols and polyglucosides;
(b9) trialkyl phosphates;
(b10) wool wax alcohols;
(b11) polysiloxane/polyalkyl polyether copolymers and corresponding derivatives thereof;
(b12) mixed esters of pentaerythritol, fatty acids, citric acid and fatty alcohols;
(b13) polyalkylene glycols,
and
(c) 0 to 40% by weight of polyols,
with the proviso that the quantities add up to 100% by weight.

2. Pearlizing concentrates as claimed in claim 1, **characterized in that** they additionally contain emulsifiers of the alkyl ether sulfate type.

3. Pearlizing concentrates as claimed in claims 1 and 2, **characterized in that** they contain 0.1 to 40% by weight of glycerol, 1,2-propylene glycol, butylene glycol, hexylene glycol and/or polyethylene glycols with an average molecular weight of 100 to 1,000 dalton as component (c).

4. A process for the production of the pearlizing concentrates claimed in claim 1, **characterized in that** a mixture of components (a), (b) and (c) is prepared, heated to a temperature 1 to 30°C above the melting point of the mixture, mixed with the necessary quantity of water having substantially the same temperature and then cooled to room temperature.

5. A process for the production of opaque and pearlescent liquid water-containing preparations of water-soluble surface-active substances, in which the pearlizing concentrates claimed in claims 1 to 3 are added to the clear aqueous preparations at 0 to 40°C in a quantity of 0.5 to 40% by weight, based on the preparation, and distributed therein by stirring.

6. The use of 12-hydroxystearic acid or salts thereof and/or 12-hydroxystearyl alcohol as pearlizing waxes for the production of surface-active formulations.

## Revendications

1. Concentrés aqueux à lustre nacré, contenant (par rapport à la partie anhydre) :
(a) de 1 à 99,9 % en poids d'acide 12-hydroxystéarique ou de ses sels et/ou d'alcool 12-hydroxystéarylique ;
(b) de 0,1 à 90 % en poids d'émulsionnants sélectionnés dans le groupe formé de :
(b1) produits d'addition de 2 à 30 moles d'oxyde d'éthylène et/ou 0 à 5 moles d'oxyde de propylène à des alcools gras linéaires comportant de 8 à 22 atomes C, à des acides gras comportant de 12 à 22 atomes C, à des alkylphénols comportant de 8 à 15 atomes C dans le groupe alkyle et à des triglycérides ;
(b2) mono- et diesters d'acides gras en C_{12/18} de produits d'addition de 1 à 30 molécules d'oxyde d'éthylène à la glycérine ;
(b3) mono- et diesters de glycérine et mono- et diesters de sorbitane d'acides gras saturés et non saturés comportant de 6 à 22 atomes de carbone, et leurs produits d'addition d'oxyde d'éthylène ;
(b4) mono- et oligoglycosides d'alkyle comportant de 8 à 22 atomes de carbone dans le reste alkyle, et leurs analogues éthoxylés ;
(b5) produits d'addition de 15 à 60 moles d'oxyde d'éthylène à l'huile de ricin et/ou à l'huile de ricin durcie ;
(b6) esters de polyol ;
(b7) produits d'addition de 2 à 15 moles d'oxyde d'éthylène à l'huile de ricin et/ou à l'huile de ricin durcie ;
(b8) esters partiels à base d'acides gras en C_{12/22} linéaires, ramifiés, non saturés ou saturés, d'acide ricinolique, ainsi que d'acide 12-hydroxystéarique, et de glycérine, polyglycérine, pentaérythrite, dipentaérythrite, alcools de sucre et polyglucosides ;
(b9) phosphates de trialkyle ;
(b10) alcools de cire de laine ;
(b11) copolymères de polysiloxane-polyalkyle-polyéther ou dérivés correspondants ;
(b12) esters mixtes de pentaérythrite, acides gras, acide citrique et alcools gras ;
(b13) polyalkylèneglycols ;
(c) de 0 à 40 % de poids, de polyols ;
en précisant que les indications de quantités se complètent de manière à donner 100 % en poids.

2. Concentrés à lustre nacré selon la revendication 1,
**caractérisés en ce qu'**
ils contiennent en outre des émulsionnants du type des éther sulfates d'alkyle.

3. Concentrés à lustre nacré selon les revendications 1 à 2,
**caractérisés en ce qu'**
ils contiennent comme composant (c) de 0,1 à 40 % en poids de glycérine, de 1,2-propylèneglycol, de butylèneglycol, d'hexylèneglycol et/ou de polyéthylèneglycols ayant un poids moléculaire moyen compris entre 100 et 1000 Dalton.

4. Procédé de production de concentrés à lustre nacré selon la revendication 1,
**caractérisé en ce qu'**
on prépare un mélange des composants (a), (b) et (c), on le chauffe à une température de 1 à 30°C au-dessus de son point de fusion, on le mélange avec la quantité requise d'eau se trouvant à peu près à la même température, et on le refroidit ensuite jusqu'à la température ambiante.

5. Procédé de production de préparations aqueuses liquides, troubles et à lustre nacré, de substances tensioactives solubles dans l'eau,
selon lequel
on ajoute aux préparations aqueuses limpides, à une température de 0 à 40°C, des concentrés à lustre nacré selon les revendications 1 à 3 en quantité de 0,5 à 40 % de la préparation, et on les y répartit sous agitation.

6. Utilisation d'acide 12-hydroxystéarique ou de ses sels et/ou d'alcool 12-hydroxystéarylique comme cires à lustre nacré en vue de la production de préparations tensioactives.
